# EUROPEAN PATENT SPECIFICATION

(11) **EP 3 231 392 B1**
(45) Date of publication and mention of the grant of the patent: **07.08.2019**
(21) Application number: 15870076.5
(22) Date of filing: 18.12.2015
(51) Int. Cl.: A61F 2/06, A61F 2/24

(54) **SUBSTRATE FOR FORMING ARTIFICIAL VALVE AND ARTIFICIAL VALVE**
SUBSTRAT ZUR HERSTELLUNG EINES KÜNSTLICHEN VENTILS SOWIE KÜNSTLICHES VENTIL
SUBSTRAT POUR FORMER UNE VALVULE ARTIFICIELLE, ET VALVULE ARTIFICIELLE

(30) Priority: 19.12.2014 JP 2014256901
(43) Date of publication of application: 18.10.2017
(73) Proprietor: National Cerebral and Cardiovascular Center, Suita, Osaka 565-8565 (JP)
(72) Inventor: NAKAYAMA, Yasuhide, Suita-shi, Osaka 565-8565 (JP); OIE, Tomonori, Suita-shi, Osaka 565-8565 (JP)
(74) Representative: Abel & Imray
(86) International application number: PCT/JP2015/085460
(87) International publication number: WO 2016/098877

(56) References cited:
- WO-A1-2013/035864
- FR-A1- 2 399 832
- JP-A- 2012 135 406
- JP-A- 2013 240 306

## Description

### Technical Field

The present invention is defined by claim 1 and relates to a substrate for forming an artificial valve that is used for forming, by connective tissue, an artificial valve having a plurality of leaflets that bulge in a radially inward direction from an outer pipe section, and also relates to an artificial valve.

### Background Art

Many studies have been conducted regarding regenerative medicine for regenerating cells, tissue, or organs damaged by disease or accidents with artificial materials or cells.

Normally, a human body has a self-defense mechanism, and when a foreign material such as a thorn enters into a shallow position in the body, the mechanism tries to push the foreign material out of the body. Further, it is known that when a foreign material enters into a deep position in the body, fibroblasts gradually accumulate around the foreign material to form a capsule of a connective tissue structure mainly composed of fibroblasts and collagen to cover the foreign material so as to isolate the foreign material inside the body. As technology for forming biologically derived tissue from living cells by utilizing the latter self-defense reaction, several kinds of technology have been reported that form a connective tissue body by implanting a substrate as a foreign material in vivo (see Patent Literatures 1 to 3).

In addition, Patent Literature 4 discloses a substrate for forming a valved artificial blood vessel in which a leaflet forming section that forms a leaflet of a valved artificial blood vessel is set in a gap between first and second columns forming tubular sections of the valved artificial blood vessel and bulge bodies that engage with an outer peripheral side of the first and second columns and form an ampulla of an artificial blood vessel.

In addition, Patent Literature 5 discloses a substrate for forming an artificial valve which is a substrate that is placed in an environment in which a biological tissue material is present to form connective tissue on a substrate surface, and which is for forming an artificial valve having a plurality of leaflets that bulge in a radially inward direction from an outer pipe section, comprising:
a columnar substrate main body, a plurality of recesses formed in an outer peripheral surface of the substrate main body, a cover substrate that covers the recesses to define a plurality of leaflet forming spaces for forming the leaflets, and an entry opening that is defined between an edge of the cover substrate and an outer edge of the recesses and that allows connective tissue to enter the leaflet forming spaces.

### Citation List

### Patent Literature

Patent Literature 1: Japanese Patent Laid-Open No. 2007-312821
Patent Literature 2: Japanese Patent Laid-Open No. 2008-237896
Patent Literature 3: Japanese Patent Laid-Open No. 2010-094476
Patent Literature 4: Japanese Patent Laid-Open No. 2012-105860 (claim 1)
Patent Literature 5: Japanese Patent Laid-Open No. 2012-135406

### Summary of Invention

### Technical Problem

However, in the substrate described in Patent Literature 4, because a leaflet forming section is set in a gap between first and second columns and bulge bodies, it is necessary to cause connective tissue to enter into the leaflet forming section from a narrow opening between an end of the bulge bodies and the outer peripheral surface of the first and second columns, and consequently the time required to form a leaflet is liable to increase by a corresponding amount.

An object of the present invention is to provide a substrate for forming an artificial valve that allows connective tissue to easily enter a leaflet forming space and can thus shorten the time required for formation of a leaflet, and to also provide an artificial valve.

### Solution to Problem

To achieve the above object, a substrate for forming an artificial valve according to the present invention is a substrate which is placed in an environment in which a biological tissue material is present to form connective tissue on a substrate surface, and which is for forming an artificial valve having a plurality of leaflets that bulge in a radially inward direction from an outer pipe section, including: a columnar substrate main body, a plurality of recesses formed in an outer peripheral surface of the substrate main body, a cover substrate that covers the recesses to define a plurality of leaflet forming spaces for forming the leaflets, and an entry opening that is defined between an edge of the cover substrate and an outer edge of the recesses and that allows connective tissue to enter the leaflet forming spaces. In addition, a slit that allows a leaflet forming space and outside of the substrate to communicate is formed in the cover substrate, the cover substrate is drawably mounted in a substrate central axis direction on the substrate main body, and the slit is formed parallel to the substrate central axis and to a length that extends to the edge of the cover substrate.

According to the above described configuration, since a leaflet forming space is defined by covering a recess in a substrate main body with a cover substrate, and an entry opening is defined between an edge of the cover substrate and an outer edge of the recess, while allowing connective tissue to enter from the entry opening to form a leaflet in the leaflet forming space, a connecting section between an end of the leaflet and an outer pipe section of an artificial valve can be formed at the entry opening. By this means, an artificial valve having a structure in which ends of leaflets are supported by an outer pipe section can be formed, and a configuration can be achieved in which a passage is opened and closed as a result of the leaflets receiving the pressure of a fluid and being deflected in a radial direction.

Further, since a slit is formed in the cover substrate, connective tissue can be allowed to enter from the slit also and not only from the narrow entry opening, and thus allow connective tissue to enter a leaflet forming space is made easier by a corresponding amount, and the time required for formation of a leaflet can be shortened.

Furthermore, although connective tissue that is formed inside a slit connects with an intermediate section of a leaflet at the outer pipe section, by cutting this connection there is no hindrance to deflection of the leaflet, and furthermore cutting the connection results in formation of a ridge that protrudes from the leaflet and that functions as a reinforcement section that reinforces the leaflet. In addition, although the connecting section between the leaflet and the outer pipe section is a site that, of the entire leaflet that repeats deflection, is damaged comparatively easily, because the slit extends as far as the edge of the cover substrate, the ridge that also reinforces the connecting section can be formed in the leaflet. Note that, a configuration may also be adopted in which the slit is formed only in the vicinity of the edge of the cover substrate so as to reinforce, of the entire leaflet, the vicinity of the connecting section, and is not formed over the entire length of the cover substrate.

Further, since the slit is formed parallel to the substrate central axis and to a length that extends to the edge of the cover substrate, the cover substrate can be drawn off without destroying connective tissue inside the slit, and the artificial valve can be easily separated from the substrate. That is, although if a hole-shaped slit or a slit that intersects with the drawing direction is formed, the connective tissue inside the slit will be destroyed when drawing off the cover substrate, by forming the slit in a shape that extends to the edge in parallel with the drawing direction, the cover substrate can be drawn off without destroying connective tissue inside the slit.

In this case, although the term "connective tissue" normally refers to tissue having collagen as a main component that is tissue which is formed in vivo, as used in the present description and claims the term "connective tissue" is a concept that also includes the tissue in a case where tissue that corresponds to connective tissue formed in vivo is formed in an ex-vivo environment.

Further, the term "biological tissue material" refers to a substance required for forming a desired biologically derived tissue and, as examples thereof, animal cells such as fibroblasts, smooth muscle cells, endothelial cells, stem cells, ES cells and iPS cells, various proteins (collagen, elastin), saccharides such as hyaluronic acid, and other various physiologically active substances present in a living body such as cell growth factors and cytokine may be mentioned. The term "biological tissue material" includes materials derived from mammals such as humans, dogs, cows, pigs, goats and sheep, and from birds, fish, and other animals, as well as artificial materials that are equivalent thereto.

Further, the term "environment in which a biological tissue material is present" refers to the inside of a living body (for example, subcutaneously implanted in the limbs, lumber, back, or abdomen; or intraperitoneally implanted) of an animal (a mammal such as a human, dog, cow, pig, goat and sheep, as well as birds, fish and other animals), or to an artificial environment containing the biological tissue material that is outside of a living body of an animal.

In the present description and claims, the term "slit" refers to a slit having a slit length that is greater than a slit width, and a slit having a slit length that is greater than twice the size of the slit width is more preferable. Note that, although the slit is set so as to have a slit width that can easily allow connective tissue to enter the slit, and to have a slit length that is greater than the slit width, preferably two or more times greater than the size of the slit width, an upper limit of the slit length is determined according to the size or strength of the substrate.

Since the slit length of the slit is sufficiently long in comparison to the slit width, while setting the slit width to a narrow width and securing an adequate remaining area of the cover substrate, the occurrence of a situation in which the slit is blocked at an early stage by connective tissue formed at a rim section can be prevented and connective tissue can be easily caused to enter into a leaflet forming space.

That is, although it is also conceivable to form a notch that has a short length at an end of the cover substrate instead of a slit, in such a case there is a concern that, due to connective tissue being formed at a rim section of the notch, the notch will be blocked from three directions by the connective tissue itself that is formed around the whole circumference of the notch excluding an opening section thereof, and thus entry of connective tissue into the leaflet forming space will be inhibited. In contrast, in a case where a slit is formed in the cover substrate, although connective tissue is also formed at the rim section thereof, since the connective tissue formed at the end of the slit does not seep out as far as a site that is separated from the slit end, the occurrence of a situation in which the entire slit is blocked at an early stage can be prevented, and connective tissue can be easily allowed to enter the leaflet forming space.

In addition, an outer fence which allows communication between inside and outside may be provided on an outer circumferential side of the substrate main body and the cover substrate.

According to this configuration, since an outer fence is provided on the outer circumferential side of the substrate main body and the cover substrate, formation of connective tissue is promoted at the outer fence and an outer pipe section of an artificial valve that is formed on the outer circumferential side of the substrate main body and the cover substrate can be thickened and the quality such as the strength thereof can be enhanced. Moreover, as described above, in the substrate for forming an artificial valve of the present invention, because a slit is formed in the cover substrate of the substrate to allow connective tissue to easily enter a leaflet forming space, the occurrence of a situation in which formation of a leaflet is delayed can be prevented while providing the outer fence on the outer circumferential side of the cover substrate.

In addition, a stent that is to be embedded in the outer pipe section may be arranged on the outer circumferential side of the substrate main body and the cover substrate.

According to this configuration, since a stent is arranged on the outer circumferential side of the substrate main body and the cover substrate, and the stent is embedded in the outer pipe section of the artificial valve, for example, the artificial valve can be placed at a site where the artificial valve is to be transplanted by increasing the diameter of the outer pipe section at the transplantation site. Moreover, as described above, since a slit is formed in the cover substrate, formation of a leaflet is not delayed by the presence of the stent.

In addition, at least an outer peripheral surface of the substrate main body and the cover substrate may be formed by a high polymer material, and a metal connecting shaft that spans the entry opening in the substrate central axis direction and connects the substrate main body and the cover substrate may be drawably provided in the substrate central axis direction.

According to this configuration, since a metal connecting shaft spans the entry opening and connects the substrate main body and the cover substrate, the end of the cover substrate can be supported by the metal connecting shaft and thus a decrease in the rigidity of the cover substrate due to the presence of the slit can be suppressed. Moreover, since the metal connecting shaft is drawably provided in the substrate central axis direction, when separating the artificial valve from the substrate, the cover substrate is not inhibited from being drawn off by the metal connecting shaft.

Further, since the outer peripheral surface of the substrate main body and the cover substrate is formed by a high polymer material having a proper degree of biocompatibility and inflammatory properties, formation of connective tissue can be promoted on the surface thereof. Moreover, since the member that spans the entry opening is the metal connecting shaft on which it is comparatively difficult for connective tissue to form, it is difficult for formation of connective tissue to be promoted at the entry opening, and blocking of the entry opening by connective tissue at an early stage can be prevented.

Furthermore, the present invention provides a method for producing an artificial valve using the above described substrate for forming an artificial valve.

That is, a production method according to the present invention includes: a placement step of placing a substrate for forming an artificial valve in an environment in which a biological tissue material is present, a formation step of forming connective tissue around the substrate for forming an artificial valve and in a leaflet forming space, a taking-out step of taking out the substrate for forming an artificial valve that is covered with connective tissue from the environment, and a separation step of separating connective tissue from the substrate for forming an artificial valve as a tubular connective tissue body including an outer pipe section and a leaflet; wherein: in the separation step, after removing connective tissue at both ends of the substrate for forming an artificial valve, while peeling off the tubular connective tissue body, the cover substrate is drawn from the substrate main body in a substrate central axis direction, and the substrate main body and the cover substrate are taken out from inside the tubular connective tissue body; the production method further including, after the separation step, a cutting step of cutting connective tissue that is formed inside the slit of the cover substrate and detaching an outer surface of a leaflet from an inner surface of the outer pipe section.

According to this configuration, the same effects can be obtained as the effects achieved by adopting the configuration of the substrate for forming an artificial valve as described above. In addition, since a cutting step of cutting connective tissue that is inside the slit of the cover substrate is provided after a separation step of drawing out the cover substrate and separating a tubular connective tissue body, it is not necessary to insert a cutting tool into a gap between the substrate and the connective tissue on the surface to cut the connective tissue inside the slit, as in a case where the cutting step is provided before the separation step, and thus formation of an artificial valve can be facilitated.

Note that, for transplanting an artificial valve produced by the method of the present invention, although any of autologous transplantation, allotransplantation, and heterotransplantation may be performed for the transplant recipient, autologous transplantation or allotransplantation is preferable in order to prevent rejection. In addition, in the case of heterotransplantation, it is preferable to perform a known immunogen removal procedure such as decellularization to avoid rejection.

The present invention further provides an artificial valve that is composed of connective tissue and that includes an outer pipe section, and a plurality of leaflets which expand in a radially inward direction from the outer pipe section and which are capable of opening and closing the outer pipe section in a passage direction, wherein a ridge that extends to a connecting section with the outer pipe section is formed in the leaflets.

According to this configuration, because a ridge is formed in the leaflets, the leaflets can be reinforced by this means, and in addition, although the connecting section between the leaflet that repeats deflection and the outer pipe section that is fixed is a site that, with respect to the entire artificial valve, is comparatively easily damaged, because the ridges of the leaflets are formed so as to extend to the connecting section, damage of the connecting section between the leaflet and the outer pipe section can be prevented.

### Advantageous Effects of Invention

As described above, according to the present invention, since a slit is formed in a cover substrate that covers a recess in a substrate main body to define a leaflet forming space, in addition to entering from an entry opening between an edge of the cover substrate and the substrate main body, connective tissue can also be allowed to enter the leaflet forming space from the slit, and thus a time period required to form an artificial valve having a leaflet can be shortened.

Further, since the slit extends to the edge of the cover substrate, connective tissue within the slit constitutes a ridge that extends to a connecting section between a leaflet and an outer pipe section, and thus the connecting section that is damaged comparatively easily can be reinforced, and moreover, since the slit is formed parallel to the substrate central axis, the cover substrate can be drawn off and the artificial valve can be separated therefrom without destroying connective tissue within the slit.

### Brief Description of Drawings

Figure 1 is a perspective view of a substrate for forming an artificial valve according to the present invention (first embodiment).
Figure 2 is a cross-sectional view along a section A-A in Figure 1 (transverse sectional view).
Figure 3 is a cross-sectional view along a section B-B in Figure 2 (longitudinal sectional view).
Figure 4 is a view for describing procedures for producing an artificial valve, in which (a-1) to (a-4) are longitudinal sectional views, and (b-1) to (b-4) are transverse sectional views.
Figure 5 is a perspective view of an artificial valve.
Figure 6 is a perspective view of a substrate for forming an artificial valve (second embodiment).
Figure 7 is a perspective view of a substrate for forming an artificial valve (third embodiment).

### Description of Embodiments

Hereunder, first to third embodiments of a substrate for forming an artificial valve as well as an artificial valve according to the present invention are described using the drawings.

### [First Embodiment]

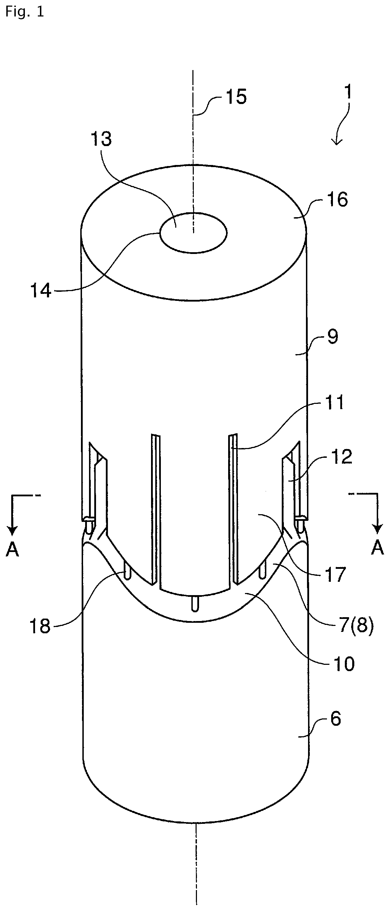

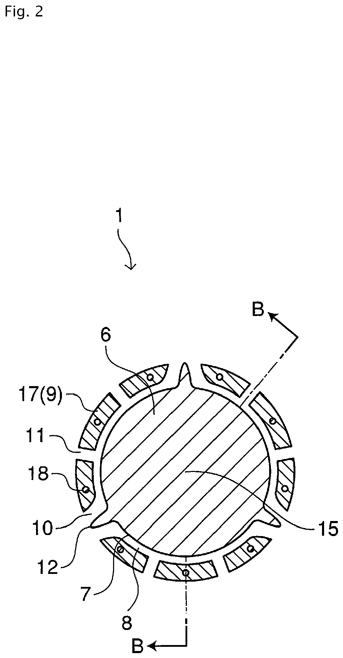

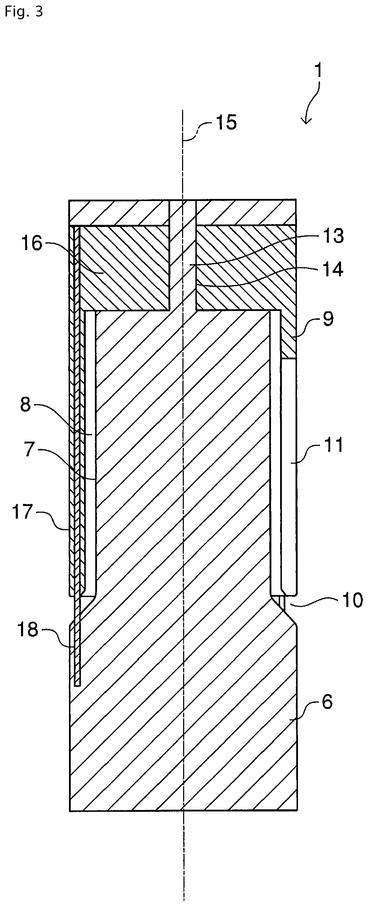

As illustrated in Figure 1 to Figure 3, a substrate 1 for forming an artificial valve is a substrate for forming an artificial valve 5 having a plurality of leaflets 4 that expand in a radially inward direction from an outer pipe section 3 and is a substrate which is placed in an environment in which a biological tissue material is present to form connective tissue 2 on the substrate surface. The substrate 1 for forming an artificial valve includes: a columnar substrate main body 6; a plurality of recesses 7 that are formed in the outer peripheral surface of the substrate main body 6; a cover substrate 9 that covers the recesses 7 to define a plurality of leaflet forming spaces 8 for forming the leaflets 4; an entry opening 10 that is defined between an edge of the cover substrate 9 and an outer edge of the recesses 7 and that allows connective tissue 2 to enter the leaflet forming spaces 8; and slits 11 that are formed in the cover substrate 9 and allow the leaflet forming spaces 8 and the outside of the substrate to communicate.

The substrate main body 6 is, for example, formed in a columnar shape and is made from a high polymer material such as acrylic resin. Three recesses 7 are arranged in parallel in the circumferential direction on the outer peripheral surface on one end side of the substrate main body 6. Each recess 7 has a shape which has a constant depth across substantially the whole area, and in which an outer edge is expanded in a circular arc shape toward the other end side, with adjacent recesses 7 being separated by partition walls 12.

A fitting protrusion 13 is formed at one end of the substrate main body 6. By fitting the fitting protrusion 13 into a fitting hole 14 of the cover substrate 9, the cover substrate 9 is drawably mounted in a direction that is parallel to a substrate central axis 15.

The cover substrate 9 is, for example, formed in a cylindrical shape and is made from a high polymer material such as acrylic resin. One end of the cover substrate 9 is blocked by a disk-like end plate 16 having the fitting hole 14 at the center thereof. At the other end side of the cover substrate 9, three cover sections 17 are formed that enter between the partition walls 12 of the substrate main body 6 to cover the recesses 7. The outer surface of the cover substrate 9 is set so that, in a state in which the cover substrate 9 is mounted on the substrate body 6, the outer surface is flush with a part other than the recesses 7 of the outer peripheral surface of the substrate main body 6, so as to form the outer pipe section 3 of the intended artificial valve 5 on the outer circumferential side thereof.

The cover section 17 is formed in a shape in which an edge is bulged in a circular arc shape toward the other end side, and leaflet forming spaces 8 for forming leaflets 4 of the artificial valve 5 are defined between the bottom face of the respective recesses 7 and the cover section 17. An entry opening 10 for the connective tissue 2 to enter the leaflet forming space 8 is defined between the edge of the cover section 17, the outer edge of the recesses 7 and the partition wall 12.

The slit 11 is set to be parallel to the substrate central axis 15 and to have a length that extends to the edge of the cover section 17. In each cover section 17, two slits 11 are arranged in parallel in the circumferential direction of the substrate over substantially the entire length of the leaflet forming space 8 to allow the connective tissue 2 to enter the leaflet forming space 8. Each of the slits 11, for example, is set to have a slit width of 0.1 mm or more that enables the entry of connective tissue, and to have a slit length that is longer than the slit width.

In the cover section 17 that is divided into narrow widths by the slits 11, the respective tip end sections are connected to the substrate main body 6 through metal connecting shafts 18 that span the entry opening 10 in a parallel direction to the substrate central axis 15, thereby suppressing a decrease in the rigidity of the cover section 17 due to the slits 11 and preventing excessive deformation of the cover section 17.

One end side of each metal connecting shaft 18 is inserted into an insertion hole that is formed over substantially the entire length of the cover substrate 9, and the other end side is inserted into an insertion hole formed from the vicinity of the outer edge of the corresponding recess 7 to the proximity thereof in the substrate main body 6. When drawing the cover substrate 9 out from the substrate body 6, the metal connecting shaft 18 does not obstruct the drawing of the cover substrate 9 with respect to the substrate body 6, and the other end side thereof is drawn out from the insertion hole in the substrate body 6 in a parallel direction to the substrate central axis 15.

As the material of the substrate 1 for forming an artificial valve, a resin having strength (hardness) such that the resin is not significantly deformed when implanted in a living body, having chemical stability as well as resistance to a load such as sterilization, and contains no or little eluate that stimulates the living body is preferable, and, as described above, acrylic resin or the like can be mentioned as an example thereof, although the material is not limited thereto.

The metal connecting shaft 18 is made from a metallic material around which it is comparatively difficult for the connective tissue 2 to be formed. The metal connecting shaft 18 spans the entry opening 10 and, while connecting the cover section 17 to the substrate main body 6 to suppress deformation of the cover section 17, can delay the occurrence of a situation in which the connective tissue 2 forms around the metal connecting shaft 18 and blocks the entry opening 10. A metal that does not easily rust is adopted as the metal that constitutes the metal connecting shaft 18, and a metallic material such as stainless steel, or titanium, cobalt, chromium or nickel-titanium alloy can be exemplified as the metal.

Next, a method for producing an artificial valve using the substrate 1 for forming an artificial valve that is described above will be described.

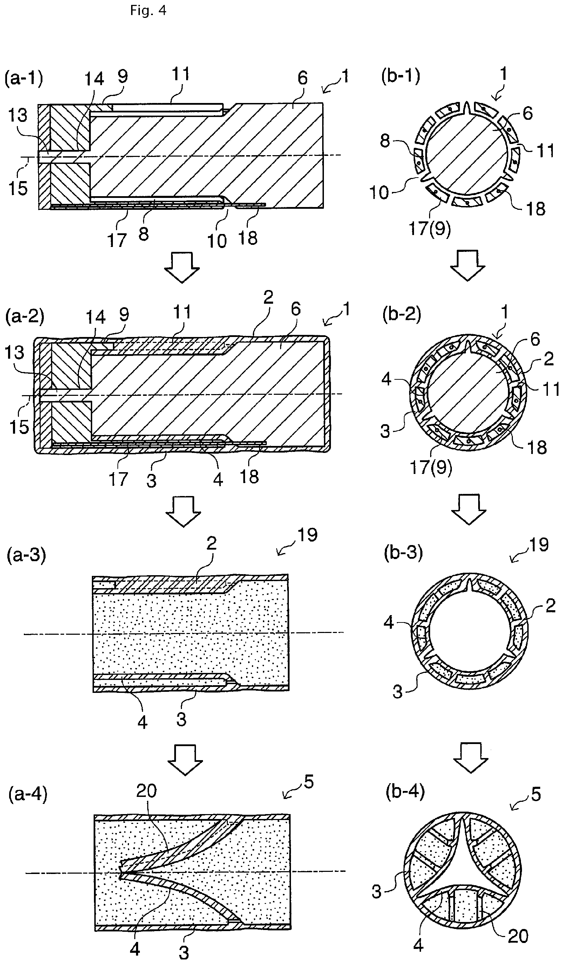

As illustrated in Figure 4, this production method includes: a "placement step" of placing the substrate 1 for forming an artificial valve in an environment in which a biological tissue material is present; a "formation step" of forming the connective tissue 2 around the substrate 1 for forming an artificial valve and in the leaflet forming space 8; a "taking-out step" of taking out the substrate 1 for forming an artificial valve that is covered by the connective tissue 2 from the environment; a "separation step" of separating the connective tissue 2 from the substrate 1 for forming an artificial valve as a tubular connective tissue structure 19 including the outer pipe section 3 and the leaflets 4; and a "cutting step" of cutting the connective tissue 2 that is formed inside the slits 11 of the cover substrate 9, and detaching the outer surface of the leaflets 4 from the inner surface of the outer pipe section 3.

### <Placement Step>

The substrate 1 for forming an artificial valve is placed in an environment in which a biological tissue material is present (Figure 4(a-1), (b-1)). Examples of an environment which the term "environment in which a biological tissue material is present" refers to include the inside of a living body of an animal (for example, subcutaneous and/or intraperitoneal implantation) or an artificial environment such as a solution in which a body tissue material is suspended that is outside the living body of an animal. Examples of the biological tissue material that can be used include: materials derived from mammals such as a human, dog, cow, pig, goat, rabbit, and sheep; materials derived from birds, fish, and other animals; and artificial materials.

In the case of implanting the substrate 1 for forming an artificial valve in an animal, the substrate is implanted with a minimum incision under sufficient anesthesia, and a wound is closed with a suture after the implantation. As a site for implanting the substrate 1 for forming an artificial valve, for example, the inside of an abdominal cavity having a capacity for receiving the substrate 1 for forming an artificial valve, or a subcutaneous region of the limbs, shoulder, back, or abdomen or the like is preferable. In addition, implanting is preferably performed by a minimally invasive method with minimum incision under sufficient anesthesia.

Further, when placing the substrate 1 for forming an artificial valve under an environment in which a biological tissue material is present, various cultivation conditions may be adjusted to perform cell culture in a clean environment according to a known method.

### <Formation Step>

After the placement step, as a predetermined time period elapses, the connective tissue 2 is formed around the substrate 1 for forming an artificial valve, and the connective tissue 2 also enters the leaflet forming spaces 8 from the entry opening 10 and the slits 11 (Figure 4(a-2), (b-2)). In this formation step, the connective tissue 2 is formed in the leaflet forming spaces 8 in a comparatively short time period that corresponds to a reduction in the required time period achieved by the connective tissue 2 entering from the slits 11 that have a sufficient area in addition to the entry opening 10. The connective tissue 2 is composed of extracellular matrix such as collagen and fibroblasts.

### <Taking-out Step>

After the connective tissue 2 is sufficiently formed in the leaflet forming spaces 8 through the formation step of a predetermined time period, a taking-out step of taking out the substrate 1 for forming an artificial valve that is covered with the connective tissue 2 from the environment in which a biological tissue material is present is performed.

### <Separation Step>

After removing the connective tissue 2 at both ends of the substrate 1 for forming an artificial valve, the sections from which the connective tissue 2 was removed are pinched and the cover substrate 9 is drawn out from the substrate main body 6 in a direction parallel to the substrate central axis 15, and the substrate body 6 and the cover substrate 9 are separated while peeling off the tubular connective tissue body 19.

When drawing out the cover substrate 9, the fitting protrusion 13 of the substrate main body 6 is drawn out from the fitting hole 14 of the cover substrate 9, and the other end side of the respective metal connecting shafts 18 is drawn out from the insertion hole of the substrate main body 6. Further, since the slits 11 are parallel to the substrate central axis 15 and are formed as far as the edge of the cover section 17, the cover substrate 9 can be drawn off while sliding the cover sections 17 relative to the connective tissue 2 inside the slits 11, the outer pipe section 3 and the leaflets 4, without destroying the connective tissue 2 inside the slits 11.

Thereafter, by taking out the separated substrate main body 6 and the cover substrate 9 from inside the tubular connective tissue body 19, the tubular connective tissue body 19 that includes the outer pipe section 3 and the leaflets 4 is obtained (Figure 4(a-3), (b-3)).

### <Cutting Step>

After taking out the tubular connective tissue structure 19, the connective tissue 2 that was formed inside the slits 11 of the cover substrate 9 is cut to thereby detach the outer surface of a center section of the leaflets 4 from the inner surface of the outer pipe section 3. By this means, the tubular connective tissue body 19 is formed into a shape in which the leaflets 4 on the three sides are connected to the outer pipe section 3 through the connective tissue 2 that is formed in the entry opening 10 of the substrate 1 for forming an artificial valve. Further, the artificial valve 5 that is composed of the connective tissue 2 is obtained by cutting off the front end edge of the respective leaflets 4 in a shape in which the center in the width direction thereof is bulged in the length direction so that, when the respective leaflets 4 deflect radially inward, the front end of the relevant leaflet 4 and the front end of the other leaflets 4 adequately contact (Figure 4(a-4), (b-4)).

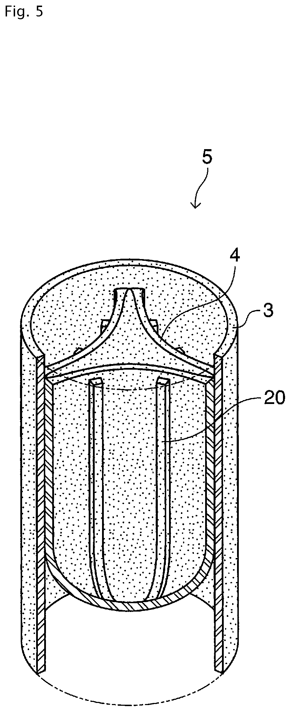

As illustrated in Figure 5, the artificial valve 5 that was produced using the substrate 1 for forming an artificial valve is an artificial valve that, for example, is to be transplanted into a blood vessel such as the aorta that exits from the heart to provide a valve function, and that is configured to open and close a passage on the inner side of the outer pipe section 3 by means of the plurality of leaflets 4 that bulge in a radially inward direction from the outer pipe section 3 being displaced inwardly and outwardly by pressure of a fluid that flows through the inside.

Each of the two side sections and the base end of the leaflet 4 are connected to the outer pipe section 3, and when the flow direction in the passage is from the base end side toward the front end side, the leaflets 4 are deflected in a radially outward direction by the flow and open the passage. On the other hand, when the flow direction in the passage is from the front end side toward the base end side, the leaflets 4 are deflected in a radially inward direction by the pressure of a fluid that attempts to enter between the leaflets 4 and the outer pipe section 3, and thereby close the passage.

In the respective leaflets 4, two ridges 20 are formed that extend from the vicinity of the front end of the leaflet 4 to a connecting section with the outer pipe section 3, and the ridges 20 reinforce the leaflet 4 that repeatedly changes position as well as a connecting section between the leaflet 4 and the outer pipe section 3. The ridges 20 are formed by, in the cutting step of the production method for producing the artificial valve 5, leaving the connective tissue 2 which is inside the slits 11 on the side of the leaflet 4 when cutting the connective tissue 2 formed inside the slits 11 to detach the leaflet 4 from the outer pipe section 3.

Note that, in a case of heterotransplanting the artificial valve 5 that was produced, an immunogen removal procedure such as decellularization, dehydration or fixing is preferably performed to prevent rejection after transplantation. Examples of the decellularization include a method such as ultrasonic treatment, surfactant treatment, eluting extracellular matrix by enzyme treatment with collagenase or the like and washing. Examples of a dehydration method include washing with a water-soluble organic solvent such as methanol, ethanol, or isopropyl alcohol. Examples of a fixing method include treatment with an aldehyde compound such as glutaraldehyde or formaldehyde.

### [Second Embodiment]

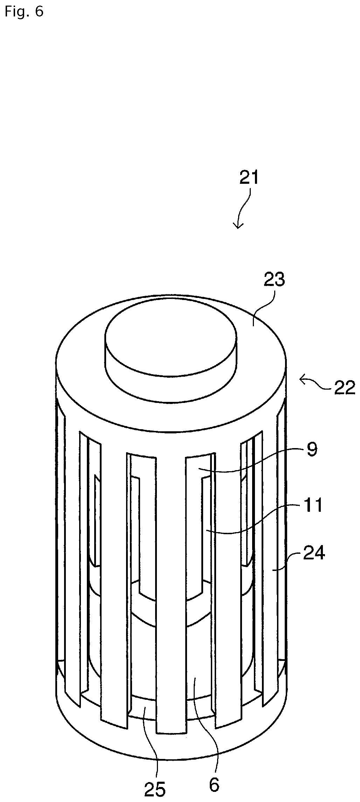

Although the second embodiment is substantially the same as the first embodiment, as illustrated in Figure 6, a substrate 21 for forming an artificial valve of the second embodiment has a structure in which an outer fence 22 through which inside and outside communicate is provided on the outer circumferential side of the substrate body 6 and the cover substrate 9.

The outer fence 22 has a structure in which a plurality of plates 24 project in the center axis direction with intervals therebetween in the circumferential direction from an outer peripheral edge of a disk-like end plate 23, in which the end plate 23 is superposed on an end face of the cover substrate 9, and the front ends of the plates 24 are engagingly provided in a flange 25 that is integrated with the substrate main body 6.

By providing the outer fence 22 and facilitating formation of connective tissue 2 around the respective plates 24 of the outer fence 22, connective tissue 2 having a thickness that envelops the outer fence 22 can be formed on the outer circumferential side of the substrate main body 6 and the cover substrate 9, and the outer pipe section 3 that is constituted by the connective tissue 2 can be thickened and the quality such as the strength can be enhanced. Furthermore, as described in the first embodiment, since the slits 11 are formed in the cover substrate 9, after allowing the connective tissue 2 to enter inside of the outer fence 22 from outside the substrate, the connective tissue 2 can further enter easily into the leaflet forming spaces 8 on the inner side of the cover substrate 9.

In a separation step during production of an artificial valve using the substrate 21 for forming an artificial valve of the present embodiment, the front ends of the plates 24 may be separated from the flange 25 to pull out the plates 24 and draw the outer fence 22 out from the outer pipe section 3. Note that the remaining configuration is the same as in the first embodiment.

### [Third Embodiment]

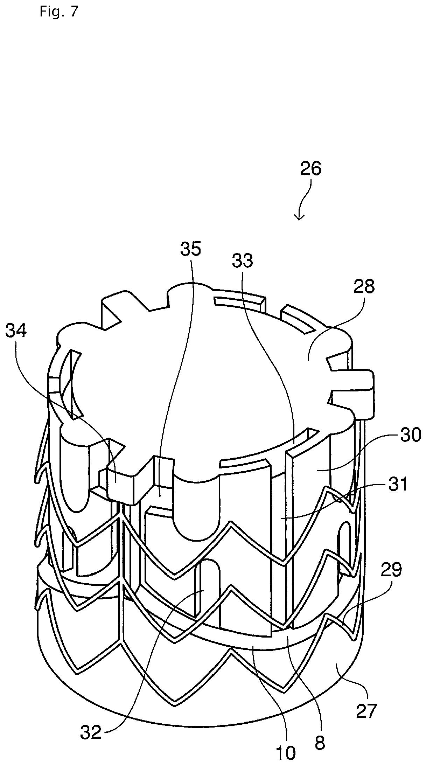

Although the third embodiment is substantially the same as the first embodiment, as illustrated in Figure 7, a substrate 26 for forming an artificial valve of the third embodiment is a substrate in which a stent 29 that is to be embedded in the outer pipe section 3 is disposed on an outer circumferential side of a substrate main body 27 and a cover substrate 28.

In a cover section 30 of the cover substrate 28, a slit 31 is formed over the entire length of the cover section 30 at the center thereof, and slits 32 that are located only in the vicinity of the entry opening 10 are formed on both sides of the cover section 30. Further, an opening 33 of the leaflet forming space 8 is formed on one end side of the cover substrate 28, and thus the configuration allows the connective tissue 2 to enter the leaflet forming space 8 from the opening 33 also, in addition to from the entry opening 10 and the slits 31 and 32.

By this means, the connective tissue 2 can be allowed to easily enter the leaflet forming space 8 from the entry opening 10, the slit 31 that extends across the entire length of the cover section 30, and the opening 33 on the one end side of the cover substrate 28. Furthermore, by cutting the connective tissue 2 that is formed inside the slits 32 that are located only in the vicinity of the entry opening 10 in addition to the slit 31 that extends across the entire length of the cover section 30, ridges can be formed that reinforce a connecting section between the leaflet 4 that repeatedly changes position and the outer pipe section 3.

The stent 29 is formed in an overall cylindrical shape having an expandable diameter by connecting a plurality of annular wires having a zig-zag shape by means of connecting members, and is configured so that by expanding the diameter of an artificial valve having the stent 29 embedded in the outer pipe section 3 at the transplantation site, the artificial valve can be placed inside a blood vessel or the like. The stent 29 is arranged on the outer circumferential side of the substrate main body 27 and the cover substrate 28 by holding a tip end of the connecting members in a stent holding section 34 of the cover substrate 28. A notch 35 is formed in the cover section 30 in the vicinity of the stent holding section 34 so that a side edge of the leaflet 4 is connected more firmly to the outer pipe section 3.

For example, a metal that does not easily rust is adopted as the metal constituting the stent 29, and a metallic material such as stainless steel, or titanium, cobalt, chromium or nickel-titanium alloy can be exemplified as the metal. Note that the remaining configuration is the same as in the first embodiment.

### Reference Signs List

- 1: Substrate for forming an artificial valve
- 2: Connective tissue
- 3: Outer pipe section
- 4: Leaflet
- 5: Artificial valve
- 6: Substrate main body
- 7: Recess
- 8: Leaflet forming space
- 9: Cover substrate
- 10: Entry opening
- 11: Slit
- 12: Partition wall
- 13: Fitting protrusion
- 14: Fitting hole
- 15: Substrate central axis
- 16: End plate
- 17: Cover section
- 18: Metal connecting shaft
- 19: Tubular connective tissue body
- 20: Ridge
- 21: Substrate for forming an artificial valve
- 22: Outer fence
- 23: End plate
- 24: Plate
- 25: Flange
- 26: Substrate for forming an artificial valve
- 27: Substrate main body
- 28: Cover substrate
- 29: Stent
- 30: Cover section
- 31: Slit
- 32: Slit
- 33: Opening
- 34: Stent holding section
- 35: Notch

## Claims

1. A substrate for forming an artificial valve(1,21,26) which is a substrate that is placed in an environment in which a biological tissue material is present to form connective tissue(2) on a substrate surface, and which is for forming an artificial valve(5) having a plurality of leaflets(4) that bulge in a radially inward direction from an outer pipe section(3), comprising:
a columnar substrate main body(6,27), a plurality of recesses(7) formed in an outer peripheral surface of the substrate main body(6,27), a cover substrate(9,28) that covers the recesses(7) to define a plurality of leaflet forming spaces(8) for forming the leaflets(4), and an entry opening(10) that is defined between an edge of the cover substrate(9,28) and an outer edge of the recesses(7) and that allows connective tissue(2) to enter the leaflet forming spaces(8);
**characterized in that** a slit(11,31,32) that allows a leaflet forming space(8) and outside of the substrate to communicate is formed in the cover substrate(9,28), the cover substrate(9,28) is drawably mounted in a substrate central axis direction on the substrate main body(6,27), and the slit(11,31,32) is formed parallel to the substrate central axis(15) and to a length that extends to the edge of the cover substrate(9,28).

2. The substrate for forming an artificial valve(21) according to claim 1, wherein an outer fence(22) which allows communication between inside and outside is provided on an outer circumferential side of the substrate main body(6) and the cover substrate(9).

3. The substrate for forming an artificial valve(26) according to claim 1 or 2, wherein a stent(29) that is to be embedded in the outer pipe section(3) is arranged on an outer circumferential side of the substrate main body(27) and the cover substrate(28).

4. The substrate for forming an artificial valve(1) according to claim 1, 2 or 3, wherein at least an outer peripheral surface of the substrate main body(6) and the cover substrate(9) is formed by a high polymer material, and a metal connecting shaft(18) that spans the entry opening(10) in the substrate central axis direction and connects the substrate main body(6) and the cover substrate(9) is drawably provided in the substrate central axis direction.

5. A production method for producing an artificial valve, comprising:
a placement step of placing a substrate for forming an artificial valve(1,21,26) according to any one of claims 1 to 4 in an environment in which a biological tissue material is present, a formation step of forming connective tissue(2) around the substrate for forming an artificial valve(1,21,26) and in a leaflet forming space(8), a taking-out step of taking out the substrate for forming an artificial valve(1,21,26) that is covered with connective tissue(2) from the environment, and a separation step of separating connective tissue(2) from the substrate for forming an artificial valve(1,21,26) as a tubular connective tissue body(19) including an outer pipe section(3) and a leaflet(4);
wherein:
in the separation step, after removing connective tissue(2) at both ends of the substrate for forming an artificial valve(1,21,26), while peeling off the tubular connective tissue body(19), the cover substrate(9,28) is drawn from the substrate main body(6,27) in a substrate central axis direction, and the substrate main body(6,27) and the cover substrate(9,28) are taken out from inside the tubular connective tissue body(19);
the production method further comprising, after the separation step, a cutting step of cutting connective tissue(2) that is formed inside the slit(11,31,32) of the cover substrate(9,28) and detaching an outer surface of a leaflet(4) from an inner surface of the outer pipe section(3).

6. An artificial valve(5) composed of connective tissue(2), comprising an outer pipe section(3), and a plurality of leaflets(4) which bulge in a radially inward direction from the outer pipe section(3) and which are capable of opening and closing the outer pipe section(3) in a passage direction, wherein a ridge(20) that extends to a connecting section with the outer pipe section(3) is formed in the leaflets(4).

## Patentansprüche

1. Substrat zum Bilden einer künstlichen Klappe (1, 21, 26), bei dem es sich um ein Substrat handelt, das in einer Umgebung angeordnet ist, in der ein biologisches Gewebematerial vorhanden ist, um Bindegewebe (2) auf einer Substratoberfläche zu bilden, und das zum Bilden einer künstlichen Klappe (5) mit einer Mehrzahl von Segeln (4) dient, die von einem äußeren Rohrbereich (3) in Richtung radial nach innen gewölbt sind, aufweisend:
einen säulenförmigen Substrathauptkörper (6, 27), eine Mehrzahl von Aussparungen (7), die in einer Außenumfangsfläche des Substrathauptkörpers (6, 27) gebildet sind, ein Abdecksubstrat (9, 28), das die Aussparungen (7) abdeckt, um eine Mehrzahl von Segelherstellungsräumen (8) zum Bilden der Segel (4) zu bilden, und eine Eintrittsöffnung (10), die zwischen einem Rand des Abdecksubstrats (9, 28) und einem äußeren Rand der Aussparungen (7) gebildet ist und die es Bindegewebe (2) ermöglicht, in die Segelherstellungsräume (8) einzutreten;
**dadurch gekennzeichnet, dass** in dem Abdecksubstrat (9, 28) ein Schlitz (11, 31, 32) gebildet ist, der eine Verbindung von einem Segelherstellungsraum (8) und der Außenseite des Substrats ermöglicht, wobei das Abdecksubstrat (9, 28) in Richtung der Substratmittelachse an dem Substrathauptkörper (6, 27) ziehbar angebracht ist und der Schlitz (11, 31, 32) parallel zu der Substratmittelachse (15) sowie auf eine Länge ausgebildet ist, die bis zu dem Rand des Abdecksubstrats (9, 28) reicht.

2. Substrat zum Bilden einer künstlichen Klappe (21) nach Anspruch 1,
wobei eine äußere Umzäunung (22), die eine Verbindung zwischen innen und außen ermöglicht, auf einer Außenumfangsseite des Substrathauptkörpers (6) und des Abdecksubstrats (9) vorgesehen ist.

3. Substrat zum Bildung einer künstlichen Klappe (26) nach Anspruch 1 oder 2,
wobei ein Stent (29), der in den äußeren Rohrbereich (3) eingebettet werden soll, auf einer Außenumfangsseite des Substrathauptkörpers (27) und des Abdecksubstrats (28) angeordnet ist.

4. Substrat zum Bilden einer künstlichen Klappe (1) nach Anspruch 1, 2 oder 3, wobei mindestens eine Außenumfangsfläche des Substrathauptkörpers (6) und des Abdecksubstrats (9) aus einem Hochpolymermaterial gebildet ist, und wobei ein Metallverbindungsschaft (18), der die Eintrittsöffnung (10) in Richtung der Substratmittelachse überspannt und den Substrathauptkörper (6) und das Abdecksubstrat (9) miteinander verbindet, in Richtung der Substratmittelachse ziehbar vorgesehen ist.

5. Herstellungsverfahren zum Herstellen einer künstlichen Klappe, das folgende Schritte aufweist:
einen Platzierungsschritt zum Platzieren eines Substrats zum Bilden einer künstlichen Klappe (1, 21, 26) nach einem der Ansprüche 1 bis 4 in einer Umgebung, in der ein biologisches Gewebematerial vorhanden ist, einen Bildungsschritt zum Bilden von Bindegewebe (2) um das Substrat zum Bilden einer künstlichen Klappe (1, 21, 26) und in einem Segelherstellungsraum (8) einen Entnahmeschritt zum Entnehmen des Substrats zum Bilden einer künstlichen Klappe (1, 21, 26), die mit Bindegewebe (2) aus der Umgebung bedeckt ist, und einen Trennungsschritt zum Trennen von Bindegewebe (2) von dem Substrat zum Bilden einer künstlichen Klappe (1, 21, 26) als einen rohrförmigen Bindegewebekörper (19), der einen äußeren Rohrbereich (3) und ein Segel (4) aufweist;
wobei:
in dem Trennungsschritt nach dem Entfernen von Bindegewebe (2) an beiden Enden des Substrats zum Bilden einer künstlichen Klappe (1, 21, 26), während der rohrförmige Bindegewebekörper (19) abgezogen wird, das Abdecksubstrat (9, 28) von dem Substrathauptkörper (6, 27) in Richtung der Substratmittelachse weggezogen wird und der Substrathauptkörper (6, 27) und das Abdecksubstrat (9, 28) aus dem Inneren des rohrförmigen Bindegewebekörpers (19) entnommen werden;
wobei das Herstellungsverfahren ferner nach dem Trennungsschritt einen Schneideschritt aufweist zum Schneiden von Bindegewebe (2), das innerhalb des Schlitzes (11, 31, 32) des Abdecksubstrats (9, 28) gebildet ist, und zum Ablösen einer Außenfläche eines Segels (4) von einer Innenfläche des äußeren Rohrbereichs (3).

6. Künstliche Klappe (5), bestehend aus Bindegewebe (2), mit einem äußeren Rohrbereich (3) und einer Mehrzahl von Segeln (4), die sich von dem äußeren Rohrbereich (3) radial in Richtung nach innen wölben und die den äußeren Rohrbereich (3) in einer Durchgangsrichtung öffnen und schließen können, wobei in den Segeln (4) eine Rippe (20) gebildet ist, die sich zu einem Verbindungsbereich mit dem äußeren Rohrbereich (3) erstreckt.

## Revendications

1. Substrat destiné à former une valve artificielle (1, 21, 26), qui est un substrat qui est placé dans un environnement dans lequel un matériau de tissu biologique est présent pour former un tissu conjonctif (2) sur une surface de substrat, et qui est destiné à former une valve artificielle (5) présentant une pluralité d'ailettes (4) qui font saillie dans une direction vers l'intérieur radialement à partir d'une section de canalisation extérieure (3), comprenant :
un corps principal de substrat en forme de colonne (6, 27), une pluralité de renfoncements (7) formés dans une surface périphérique extérieure du corps principal de substrat (6, 27), un substrat de couverture (9, 28) qui couvre les renfoncements (7) afin de définir une pluralité d'espaces de formation d'ailettes (8) pour former les ailettes (4), et une ouverture d'entrée (10) qui est définie entre un bord du substrat de couverture (9, 28), et un bord extérieur des renfoncements (7), et qui permet au tissu conjonctif (2) de pénétrer dans les espaces de formation d'ailettes (8) ;
**caractérisé en ce qu'**une fente (11, 31, 32) qui permet à un espace de formation d'ailette (8) de communiquer avec l'extérieur du substrat, est formée dans le substrat de couverture (9, 28), le substrat de couverture (9, 28) est monté de manière à pouvoir être tiré dans la direction de l'axe central du substrat sur le corps principal de substrat (6, 27), et la fente (11, 31, 32) est formée parallèle à l'axe central du substrat (15) et sur une longueur qui s'étend jusqu'au bord du substrat de couverture (9, 28).

2. Substrat destiné à former une valve artificielle (21) selon la revendication 1, où une barrière extérieure (22) qui permet une communication entre l'intérieur et l'extérieur, est prévue sur un côté circonférentiel extérieur du corps principal de substrat (6) et du substrat de couverture (9).

3. Substrat destiné à former une valve artificielle (26) selon la revendication 1 ou 2, où une endoprothèse vasculaire (29) qui doit être enfouie dans la section de canalisation extérieure (3), est agencée sur un côté circonférentiel extérieur du corps principal de substrat (27) et du substrat de couverture (28).

4. Substrat destiné à former une valve artificielle (1) selon la revendication 1, 2 ou 3, où une surface périphérique extérieure au moins du corps principal de substrat (6) et du substrat de couverture (9), est formée dans un matériau de haut polymère, et une tige de connexion métallique (18) qui enjambe l'ouverture d'entrée (10) dans la direction de l'axe central du substrat et qui connecte le corps principal de substrat (6) et le substrat de couverture (9), est prévue de manière à pouvoir être tirée dans la direction de l'axe central du substrat.

5. Procédé de production destiné à produire une valve artificielle, comprenant :
une étape de placement consistant à placer un substrat destiné à former une valve artificielle (1, 21, 26) selon l'une quelconque des revendications 1 à 4 dans un environnement dans lequel un matériau de tissu biologique est présent, une étape de formation consistant à former un tissu conjonctif (2) autour du substrat afin de former une valve artificielle (1, 21, 26), et dans un espace de formation d'ailette (8), une étape d'enlèvement consistant à enlever le substrat afin de former une valve artificielle (1, 21, 26) recouverte de tissu conjonctif (2) de l'environnement, et une étape de séparation consistant à séparer le tissu conjonctif (2) du substrat afin de former une valve artificielle (1, 21, 26) en tant que corps de tissu conjonctif tubulaire (19) comprenant une section de canalisation extérieure (3) et une ailette (4) ;
où :
dans l'étape de séparation, après avoir retiré le tissu conjonctif (2) aux deux extrémités du substrat afin de former une valve artificielle (1, 21, 26), tout en décollant le corps de tissu conjonctif tubulaire (19), le substrat de couverture (9, 28) est tiré hors du corps principal de substrat (6, 27) dans la direction de l'axe central du substrat, et le corps principal de substrat (6, 27) et le substrat de couverture (9, 28) sont sortis de l'intérieur du corps de tissu conjonctif tubulaire (19) ;
le procédé de production comprenant en outre, après l'étape de séparation, une étape de coupe consistant à couper le tissu conjonctif (2) qui est formé à l'intérieur de la fente (11, 31, 32) du substrat de couverture (9, 28), et à détacher la surface extérieure d'une ailette (4) de la surface intérieure de la section de canalisation extérieure (3).

6. Valve artificielle (5) composée d'un tissu conjonctif (2), comprenant une section de canalisation extérieure (3), et une pluralité d'ailettes (4) qui font saillie dans une direction vers l'intérieur radialement à partir de la section de canalisation extérieure (3), et qui peuvent ouvrir et fermer la section de canalisation extérieure (3) dans une direction de passage, où une arête (20) qui s'étend jusqu'à une section de connexion avec la section de canalisation extérieure (3), est formée dans les ailettes (4).
